# FASCICULE DE BREVET EUROPEEN

(11) **EP 0 773 744 B1**
(45) Date de publication et mention de la délivrance du brevet: **13.01.1999**
(21) Numéro de dépôt: 95928456.3
(22) Date de dépôt: 24.07.1995
(51) Int. Cl.: A61B 17/41

(54) **ELECTRODE FLEXIBLE POUR LE TRAITEMENT HAUTE FREQUENCE DE LA PEAU**
FLEXIBLE ELEKTRODE ZUR BEHANDLUNG DER HAUT MIT HOCHFREQUENZ
FLEXIBLE PROBE FOR HIGH FREQUENCY SKIN TREATMENT

(30) Priorité: 02.08.1994 EP 94810452
(43) Date de publication de la demande: 21.05.1997
(73) Titulaire: Bernaz, Gabriel, CH-1227 Carouge (CH)
(72) Inventeur: Bernaz, Gabriel, CH-1227 Carouge (CH)
(74) Mandataire: Munzinger, John Patrick
(86) Numéro de dépôt international: EP9502930
(87) Numéro de publication internationale: WO9603928

(56) Documents cités:
- WO-A-93/04636
- GB-A- 2 132 892

## Description

### DOMAINE TECHNIQUE

L'invention se rapporte au traitement de la peau par des moyens électriques à haute fréquence, en particulier pour l'épilation dite "définitive" ou "longue durée", ainsi que pour la repousse des cheveux.

### ETAT DE LA TECHNIQUE

WO 93/04636 décrit un procédé basé sur la constatation qu'en mélangeant un gel conducteur de type usuel pour l'application de sondes à ultrasons contre la peau avec un produit traitant, par exemple une lotion à effet d'atrophie sur les racines des poils, il était possible, par induction transcutanée à haute fréquence, de faire pénétrer le produit traitant dans les follicules pileux (pores) et ainsi d'effectuer un traitement.

Ce procédé permet ainsi d'effectuer un traitement, notamment cosmétique, de la peau, par exemple en vue d'une épilation longue durée, et permet de surcroît une application ponctuelle et efficace au niveau des follicules, sans intervention manuelle délicate. La même technique convient également pour introduire dans la peau d'autres produits cosmétiques, par exemple pour la repousse des cheveux ou contre les rides, ainsi que des produits pharmaceutiques, par exemple pour le traitement de l'acné, de la séborrhée, de plaies ou de douleurs.

Pour la mise en oeuvre du procédé, WO 93/04636 décrit en outre un appareil comprenant un organe maniable de contact avec la peau ayant un corps non-conducteur pourvu d'une surface d'appui destinée à être appliquée sur la peau. Cette surface comporte une pluralité de points conducteurs d'émission électromagnétique distincts formés par exemple par des parties exposées de spires d'un solénoïde noyé dans le corps de l'organe de contact. Ces points débouchent par des orifices de cette surface, de préférence en retrait par rapport à celle-ci, de manière que, en cours d'utilisation de l'appareil, ils puissent contacter du gel conducteur appliqué sur la peau. Ces points d'émission distincts émettent un flux d'énergie électromagnétique à haute fréquence, avantageusement un courant émetteur pur, fourni par un circuit électrique oscillant haute fréquence de puissance.

L'organe maniable de l'appareil décrit ci-dessus forme donc une sonde focalisée dont l'énergie haute fréquence assure une action ponctuelle à travers le gel chargé. En disposant les points d'émission distincts d'une manière adéquate sur la surface d'appui, on obtient une action simultanée sur tous les follicules d'une zone de la peau. Par exemple, les points d'émission distincts sont alignés sur une ou plusieurs rangées le long d'une surface d'appui oblongue de dimensions adaptées à la partie du corps à traiter. De préférence, il est prévu plusieurs organes de contact rigides interchangeables, ayant des surfaces d'appui de formes et/ou de grandeurs différentes, et éventuellement aussi un embout à aiguille interchangeable, permettant ainsi des traitements propres à toutes sortes de pilosités.

Le circuit électrique générateur de haute fréquence dudit appareil comprend avantageusement un circuit oscillant haute fréquence de puissance comportant un transistor monté en oscillateur de puissance, en combinaison avec des bobines de self inductance double spirale en carré, et une électrode permettant d'ajouter l'impédance du corps d'une personne traitée à celle des bobines de self inductance, de manière à élever la fréquence du courant émetteur lors de l'utilisation.

### EXPOSE DE L'INVENTION

La présente invention a pour objet une sonde pour l'application d'un flux d'énergie électromagnétique haute fréquence sur la peau, utilisable notamment dans le procédé décrit dans WO 93/04636 ou autre procédé nécessitant l'application d'un gel conducteur, cette sonde étant pourvue d'une surface d'appui non-conductrice, destinée à être appliquée sur la peau, cette surface présentant une pluralité de points ou zones conducteurs d'émission électromagnétique distincts formés par des parties de spires d'une bobine disposées en retrait par rapport à cette surface.

La sonde selon l'invention est caractérisée par les caractéristiques de la revandication 1.

Lors de l'utilisation de la sonde, on applique un mélange de gel/produit bio-actif traitant sur la surface de la peau à traiter et/ou dans les cavités de la surface d'appui de la sonde de manière à ce que ce mélange serve d'interface conducteur entre la surface d'appui de la sonde flexible et la peau.

La sonde flexible selon l'invention présente de nombreux avantages. Par exemple, elle peut être fabriquée aisément dans toutes les formes et dimensions souhaitées pour permettre un traitement efficace des différentes parties du corps.

Une fois appliquée sur le corps, après application préalable de gel conducteur sur la semelle de la sonde ou sur la peau, la sonde flexible n'exige aucune intervention manuelle pendant le traitement. Celui-ci peut dès lors être programmé pour une durée prédéfinie, ce qui permet une pénétration optimale du produit, d'où un traitement plus efficace. Donc, la sonde flexible se prête non seulement au traitement de grandes surfaces, mais aussi au traitement de petites surfaces.

La flexibilité de la sonde procure un bon contact, même sur les parties courbes telles que les bras et les jambes, ainsi que sur les parties creuses. On peut donc l'appeler une sonde morphologique car elle se conforme à la morphologie de la partie du corps traitée.

L'utilisation de la technique de balayage sectoriel permet le traitement de grandes surfaces avec une économie d'énergie. De plus, l'emploi de deux sondes flexibles semblables, soumises toutes deux à la technique de balayage sectoriel, permet le traitement simultané, par exemple, de deux jambes ou de deux bras.

L'emploi d'une ou plusieurs bobines aplaties dans la (ou chaque) sonde flexible permet une utilisation maximale de la surface de la bobine pour la transmission du flux électromagnétique, donc d'augmenter considérablement le nombre de points ou zones conducteurs d'émission électromagnétique. Avec la nouvelle sonde flexible, on peut prévoir des surfaces avec, par exemple, deux cents à deux mille points ou zones d'émission, voire plus, alors que, avec la sonde maniable de WO 93/04636, le nombre maximal de points d'émission utilisés est, dans la pratique, de septante-cinq, c'est-à-dire trois rangées de vingt-cinq points.

Avantageusement, la sonde comporte une surface d'appui sur ses deux faces, ce qui procure à la sonde une flexibilité accrue, améliorant ainsi sa capacité de se conformer aux parties du corps sur lesquelles elle est appliquée. De plus, chaque face peut présenter des cavités de formes et/ou de dimensions et/ou de distribution différente(s) de celles de l'autre face.

Une sonde alvéolaire, avec des cavités de grandeurs différentes sur ses deux faces, a l'avantage de permettre des traitements différents du fait que la peau est exposée à des flux d'énergie électromagnétique différents, selon la présentation différente des cavités sur les deux faces, ainsi que de permettre l'application de quantités différentes de mélange en choisissant la face utilisée, en raison des différentes grandeurs des cavités.

D'autre part, les sondes flexibles selon l'invention peuvent être fabriquées par un simple moulage avec la ou les bobines aplaties disposées en sandwich entre ledit dos et la semelle alvéolaire ou ajourée, permettant des économies considérables par rapport aux sondes maniables rigides utilisées actuellement.

Les spires de la bobine aplatie sont de préférence formées par une feuille conductrice en résine de silicone conductrice, par exemple contenant un verre fritté argenté, ou par une feuille de caoutchouc graphité conducteur. Il est également possible d'employer une impression métallique sur un support flexible adéquat.

L'invention concerne également l'utilisation de cette sonde flexible pour l'épilation longue durée et pour le traitement de la calvitie, ainsi que l'utilisation d'un mélange gel/conducteur comme interface conductrice entre la surface d'appui d'une sonde flexible et la peau, et comme moyen de transport d'un produit bio-actif pour amener celui-ci, contenu dans une solution conductrice provenant du mélange, à pénétrer dans les follicules pileux de la peau jusqu'aux racines des poils ou des cheveux.

L'invention concerne aussi un procédé de traitement cosmétique où on applique le mélange sus-mentionné dans des cavités ménagées dans la surface d'une sonde flexible susceptible de se conformer à la partie du corps contre laquelle elle est appliquée, on applique la surface de la sonde flexible recouverte de mélange sur la peau, et on applique le flux d'énergie électromagnétique de haute fréquence par les cavités de la sonde flexible.

Selon un autre aspect avantageux d'un procédé d'épilation longue durée, avant l'application du mélange, on tond les poils d'une surface de la peau à traiter, laissant le bout des poils à fleur de la surface de la peau. On applique ensuite le mélange gel/produit et le flux d'énergie électromagnétique de haute fréquence pour amener ce produit jusqu'aux racines des poils tondus, et on répète ce traitement après un temps de repos, éventuellement sans retonte, c'est à dire en ne retondant les poils que si désiré et/ou nécessaire, jusqu'à ce que les racines des poils s'atrophient complètement et, de préférence, que les poils, avec leur racine, tombent naturellement, par exemple lors du lavage.

D'autres caractéristiques et diverses variantes de l'invention sont exposées dans les revendications dépendantes.

### BREVE DESCRIPTION DES DESSINS

D'autres caractéristiques de l'invention ressortiront également à la lecture de la description qui suit, donnée à titre d'exemple, en se référant aux dessins dans lesquels :
la Figure 1 est un schéma illustrant deux sondes flexibles selon l'invention avec un circuit de contrôle;
la Figure 2 est une coupe selon la ligne II-II de la Figure 1 à plus grande échelle;
la Figure 3 est un schéma d'une exécution de l'invention, comprenant trois bobines aplaties, reliées en parallèle à un circuit de contrôle;
la Figure 4 est un schéma d'une autre forme d'éxécution de sonde flexible, à double face;
les Figures 5 et 6 sont des vues schématiques en coupe transversale et en coupe longitudinale de la sonde flexible de la Figure 4;
les Figures 5A, 6A et 6B sont des agrandissements des parties encadrées des Figures 5 et 6;
la Figure 7 est une vue en perspective photographique montrant la surface d'appui d'une sonde semblable à celle de la Figure 4, dont un coin a été coupé; et
les Figures 7A et 7B sont des agrandissements de la partie encadrée de la Figure 7, vue en recto et en verso.

### MODES DE REALISATION PREFERES

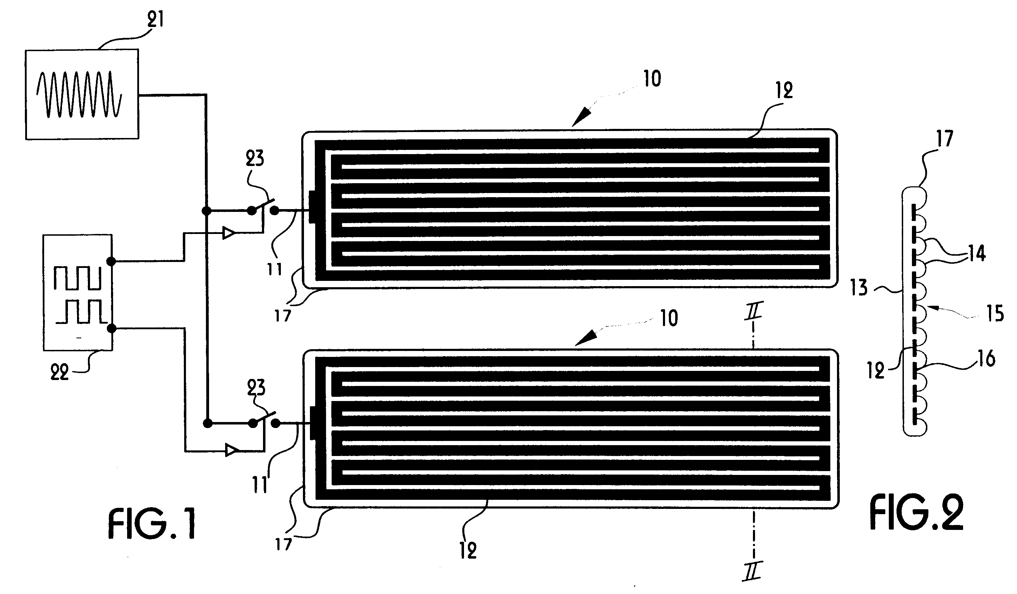

La Figure 1 représente deux sondes flexibles 10 selon l'invention, reliées en parallèle à un circuit de contrôle 21. Chaque sonde flexible 10 comporte une amenée de courant 11 alimentant une bobine aplatie 12 formée de spires aplaties, au nombre de cinq dans cet exemple. La bobine aplatie 12, de forme rectangulaire, est ménagée dans une feuille conductrice en résine de silicone, par exemple contenant du verre fritté argenté, ou tout autre feuille électriquement conductrice et flexible.

Cette bobine aplatie 12 est incorporée dans un corps non-conducteur et flexible d'une pièce, par exemple en caoutchouc, les spires aplaties de la bobine 12 étant disposées en sandwich entre un dos ou dessus 13 constitué d'une feuille non perforée, et une semelle ajourée ou dessous 14, le tout complété d'un pourtour 17 également en caoutchouc. La semelle ajourée 14 se compose, soit d'éléments s'étendant le long des parties longitudinales des spires de la bobine 12, soit d'un entrecroisement d'éléments s'étendant le long des parties longitudinales et des parties transversales des spires de la bobine 12. Par exemple, pour une sonde telle que représentée, présentant dix parties longitudinales de spires, on peut prévoir de onze à trente et une parties transversales de manière qu'il y ait entre cent et trois cents orifices 15 par lesquels les parties exposées des spires débouchent en retrait par rapport à la surface d'appui de la semelle 14, formant les points ou zones d'émission 16.

Les points ou zones d'émission 16 peuvent, par exemple, mesurer de 0.5mm à 2 mm de large (largeur exposée de la spire aplatie) et de 1mm à 10 mm de long. Le plus souvent, les points ou zones mesurent environ 1mm x 3mm, et sont disposés dans un rectangle de caoutchouc d'environ 4mm x 5mm.

Les sondes 10 sont donc des sondes morphologiques constituées chacune d'une feuille de caoutchouc flexible rectangulaire incorporant une bobine aplatie 12 dont les spires occupent la quasi-totalité de la feuille flexible, excepté son pourtour non-conducteur 17.

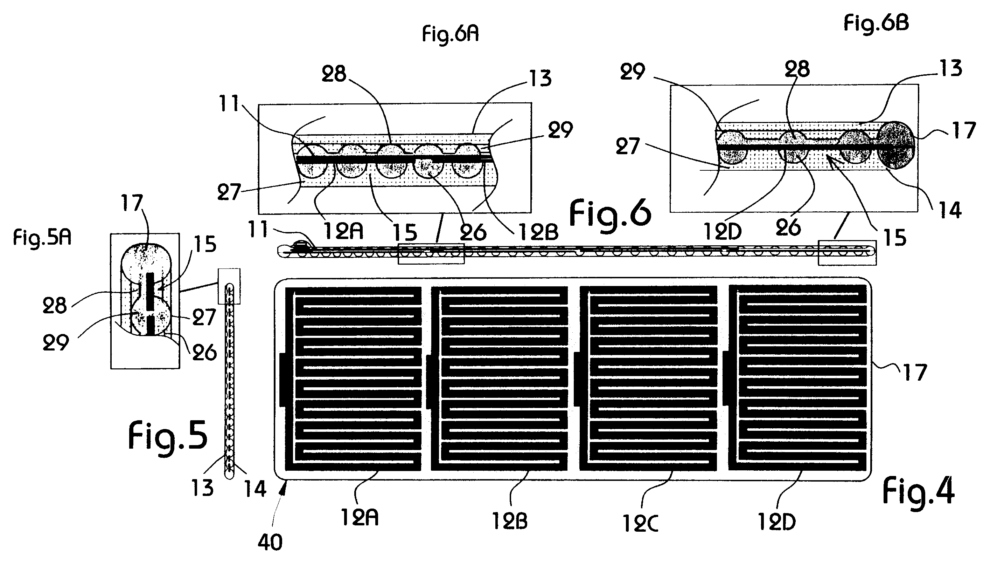

Un exemple du circuit 21 est décrit dans WO 93/04636 (voir la Figure 4). Ce circuit est contenu dans un boîtier et comprend une double alimentation stabilisée, suivie d'un circuit oscillateur délivrant en sortie un courant de faible amplitude à très haute fréquence. Par rapport aux circuits conventionnels utilisés dans ce domaine, pilotés par quartz, et qui fonctionnent à fréquence fixe, ce circuit fournit un courant haute fréquence émetteur pur, donc exempt d'harmoniques, ce qui assure une transmission efficace par les points ou zones 16 des sondes 10.

Le circuit 21 est relié aux amenées de courant 11 des sondes 10 par l'intermédiaire d'interrupteurs 23, sous contrôle d'une bascule 22 dont le fonctionnement assure une alimentation déphasée aux deux sondes 10. On peut donc utiliser les deux sondes 10 pour le traitement simultané de deux parties du corps, par exemple deux jambes ou deux bras.

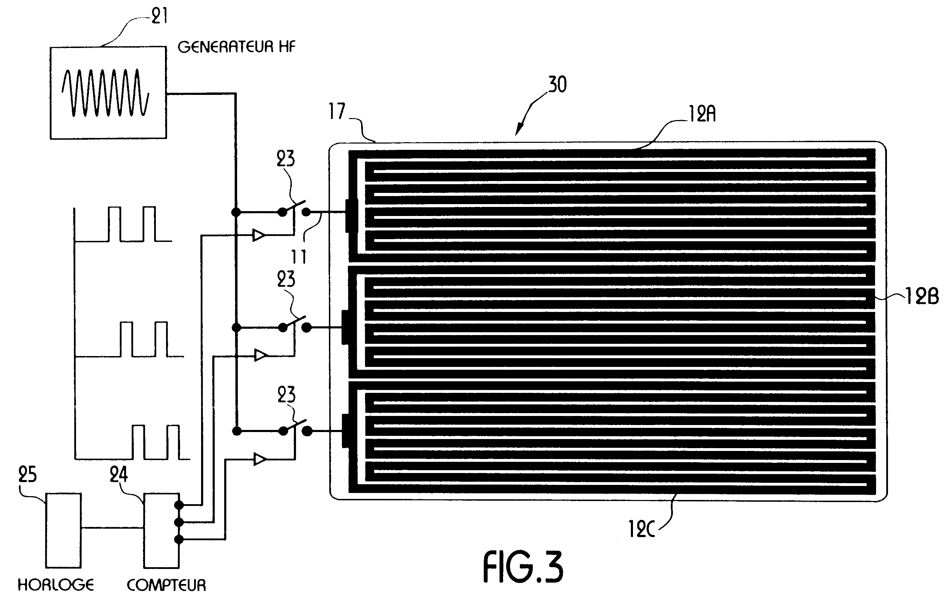

La Figure 3 représente une sonde flexible 30 comportant trois bobines aplaties 12A, 12B et 12C, disposées côte-à-côte, qui occupent la quasi-totalité de la surface de la sonde flexible 30, excepté son pourtour non-conducteur 17.

Cette sonde flexible 30 est reliée, par les amenées de courant 11 des trois bobines 12A, 12B, 12C, au circuit générateur haute fréquence 21 par l'intermédiaire de trois interrupteurs 23, sous contrôle d'un circuit compteur 24 piloté par un circuit d'horloge 25, afin d'assurer une alimentation déphasée des trois bobines 12A, 12B, 12C dite "par balayage sectoriel", c'est-à-dire le courant haute fréquence balaye successivement les trois bobines. La structure de la sonde 30, avec les bobines aplaties 12A, 12B, 12C moulées en sandwich entre un dos constitué d'une feuille non-ajourée et une semelle ajourée est semblable à ce qui a été décrit pour la Figure 2. Pour simplifier, seul le pourtour 17 est visible à la Figure 3. Il va de soi que l'utilisation de trois bobines aplaties 12A, 12B, 12C permet une augmentation substantielle du nombre de points ou zones d'émission distincts.

Il est également possible d'utiliser simultanément plusieurs sondes flexibles 30 à bobines multiples pour traiter des parties différentes du corps. Dans ce cas, il est avantageux de relier les sondes 30 au circuit générateur haute fréquence 21 par paires, une des sondes de chaque paire étant reliée à la sortie principale ou active du circuit, l'autre sonde de chaque paire étant reliée à la sortie secondaire ou indifférente du circuit à la place d'une electrode de base connectée à la personne traitée pour assurer la mise à la terre. De cette manière, on double la surface effective traitée au moyen d'un circuit donné, et il n'est plus nécessaire de disposer d'une électrode de base séparée devant être connectée à la personne traitée, par exemple en étant tenue par cette personne.

Les Figures 4, 5, 6 et 7 représentent une sonde flexible 40 comportant une feuille de caoutchouc flexible oblongue incorporant quatre bobines aplaties 12A, 12B, 12C, 12D disposées l'une à la suite de l'autre dans le sens de la longueur de la sonde 40, et qui occupent la quasi-totalité de la surface de la sonde flexible, excepté son pourtour non-conducteur 17.

Cette sonde flexible 40 est reliée par une amenée de courant 11 à un générateur haute fréquence, tel que représenté à la Figure 3. Cette amenée de courant 11 s'étend, à l'intérieur de la sonde, le long de celle-ci, par dessus les bobines aplaties 12A, 12B et 12C, et est reliée successivement (comme illustré pour la bobine 12B dans la Figure 6A) aux bobines aplaties 12A, 12B, 12C, 12D, permettant une alimentation déphasée "par balayage sectoriel" des quatre bobines. Plusieurs sondes 40 peuvent être reliées simultanément, de préférence par paires, au générateur haute fréquence, pour un traitement simultané de plusieurs parties d'un corps, ou de plusieurs personnes.

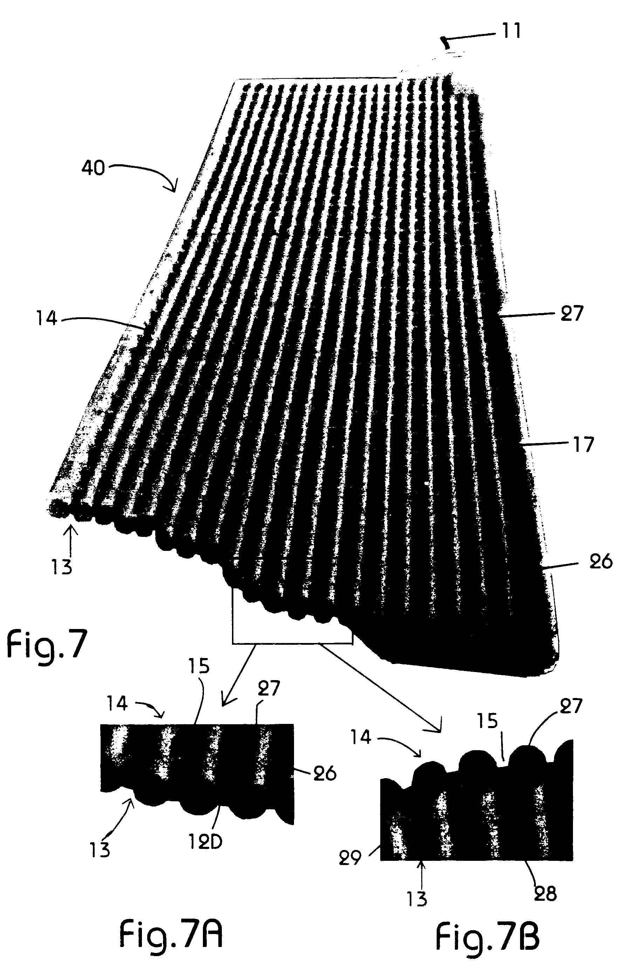

Dans l'exécution de la Figure 7, on voit que l'amenée de courant 11 est connectée à la sonde 40 par un coin renforcé du pourtour 17. Il est également possible de situer l'amenée de courant an centre de la sonde, comme dans la Figure 4.

Comme on le voit dans les Figures 5A, 6A et 6B, les bobines 12A, 12B, 12C, 12D sont moulées en sandwich entre une semelle ajourée 14 et un dessus 13 alvéolé. Par exemple, comme on le voit également dans les Figures 7, 7A et 7B, les cavités ajourées 15 du côté semelle 14 sont formées par un entrecroisement d'éléments transversaux 26 et longitudinaux 27; du côté du dessus 13, des cavités moins profondes et non-ajourées en forme de gaufrage sont formées par des éléments transversaux 28 et longitudinaux 29, et n'exposent pas les bobines. Du côté 14, les éléments longitudinaux 27 sont de la même hauteur que le pourtour 17, avec les éléments transversaux 26 légèrement en retrait; du côté 13, les éléments longitudinaux 29 et transversaux 28 sont de hauteur égale, tous deux en retrait par rapport au pourtour 17. Bien sûr, d'autres dispositions sont possibles.

Les dimensions des cavités ajourées 15 dans la semelle 14 et les cavités non-ajourées dans le dessus 13 augmentent progressivement dans le sens de la longueur de la sonde 40 (voir l'augmentation progressive de l'espacement des éléments transversaux 26/28 des Figures 6A à 6B). Par exemple, dans le cas d'une sonde 40 mesurant environ 10cm x 30cm, comportant quatre bobines à dix-huit branches tel qu'illustré à la Figure 4, on dispose de dix-huit rangées de cavités, et chaque rangée peut comporter, par exemple, de 40 à 60 cavités, ou plus, dont la longueur augmente d'environ 1mm à 3mm adjacent du côté alimentation en courant (voir la Figure 6A), jusqu'à une longueur d'environ 3mm à 5mm du côté de l'extrémité libre de la sonde 40 (voir la Figure 6B). La largeur de ces cavités peut être constante ou peut augmenter progressivement dans le sens de la largeur de la sonde 40.

Cette sonde 40 double-face, à cavités de dimensions différentes sur les deux faces, a de nombreux avantages. Un premier avantage est qu'elle présente une flexibilité accrue par rapport aux sondes 10, 30 à simple face. Un deuxième avantage, dû à l'augmentation progressive des dimensions des cavités, est qu'elle permet une distribution thermique uniforme, donc un traitement uniforme le long de la sonde. En plus, de par les profondeurs différentes des cavités des deux faces de la sonde, on peut appliquer des quantités différentes de mélange gel/produit selon le mode de traitement. Enfin, les cavités ajourées 15 côté semelle 14 permettent un contact entre les points exposés des bobines et le mélange gel/produit, tandis que les cavités non-ajourées du côté du dessus 13 sont isolées des spires des bobines, permettant une transmission atténuée du flux d'énergie éléctromagnétique pour certain traitements ne nécessitant pas une pénétration du produit jusqu'aux racines.

La disposition des bobines aplaties des sondes 10,30,40 des Figures 1, 3 et 4 n'est donnée qu'à titre d'exemple. En variante, on peut prévoir des bobines aplaties dont les spires aplaties s'étendent en travers du petit côté d'une sonde flexible rectangulaire oblongue. Le nombre de spires aplaties de chaque bobine peut être entre trois et dix, voire plus. On peut aussi prévoir des sondes flexibles d'autres formes, oblongue, carrée, circulaire, ovoïde, etc.

Le gel chargé utilisé pour le procédé de traitement est de préférence composé d'un gel conducteur non-polymérisable d'un type usuel pour le couplage ultrasonique d'électrodes sur la peau, en mélange avec un produit bio-actif. Le gel a un pH neutre et est, par exemple, à base d'un polymère de l'ester 5-bromo-5-nitro-1,3-dioxane-2-carboxyvinylique. La composition du produit bio-actif dépend de l'action souhaitée. Pour une action dépilatoire, on peut choisir un produit à effet d'atrophie progressif de la racine du poil, par exemple une lotion post-épilatoire du type utilisé d'habitude immédiatement après une épilation à la cire, ainsi que les jours suivants. De telles lotions comportent des extraits de plantes, des huiles essentielles, de l'eau déminéralisée et éventuellement d'autres composants, par exemple des polyoxyéthylènes. Ces produits, connus parfois sous la dénomination "modérateur de la repousse des poils", sont sans danger d'utilisation toxique ou autre.

Pour un traitement de la calvitie, on peut mélanger le gel avec, par exemple, du minoxydil, ou tout autre produit favorisant la repousse des cheveux. Un mélange de 50:50 vol% de gel et de minoxydil a donné des résultats satisfaisants.

Afin de ne pas compromettre les propriétés conductrices du gel, la part du produit actif ou traitant n'excédera en général pas 50%, usuellement moins de 25%, en poids du gel (% en poids = % en volume) . On peut ajouter de l'alcool, du chlorure de sodium et/ou d'autres substances pour améliorer la conductivité du produit, et/ou comme agents conservateurs.

Des tests ont démontré que l'application d'une énergie à haute fréquence sur le produit bio-actif seul ou sur le gel seul n'aboutit à aucun effet spécial, tandis qu'en mélange, on obtient une bonne pénétration du produit bio-actif entraîné par la solution conductrice provenant du gel. Il semble que la conduction de l'énergie électromagnétique et électrique suit le chemin de moindre résistance offert par le mélange du gel et de la lotion conductrice appliqué à la surface de la peau, et ensuite autour de la racine du poil ou du cheveu par la seule lotion conductrice qui pénètre dans le follicule pileux. Le gel permet un dégagement progressif de la lotion active et sa pénétration, sous l'action conjuguée des champs électromagnétique et électrique.

Le flux d'énergie électromagnétique produit par le courant haute fréquence a un excellent coefficient d'absorption dans la peau, car le système se comporte comme un émetteur-récepteur radio. A cet effet, on emploiera de préférence un courant haute fréquence émetteur pur, exempt de toute harmonique.

L'énergie du courant haute fréquence est focalisée sur la peau par les points ou zones d'émission 16 situés sur la face des sondes flexibles 10,30,40 qui est en contact avec le gel chargé. Cette énergie suit la solution conductrice et entraîne le produit actif dans le follicule; provoquant un échauffement progressif accompagné d'une dilatation du follicule pileux, ce qui favorise la pénétration du produit actif et augmente l'efficacité de l'action de ce dernier sur la racine du poil. Ceci produit un effet d'ionothermolyse intra-racine du poil, par pénétration effective de la solution conductrice et bio-active.

Au contraire des sondes maniables et rigides utilisées auparavant, la sonde flexible ou morphologique selon l'invention permet une application d'une quantité controlée de gel conducteur sur une grande surface de la peau puis l'utilisation du très grand nombre de points ou zones d'émission 16.

Il suffit donc d'appliquer le gel sur la surface alvéolée de la sonde flexible et/ou sur la peau, et de mettre en place les sondes flexibles 10, 30 et/ou 40 pour produire, après quelques minutes de traitement, un assouplissement du follicule pileux et, en cas de traitement dépilatoire, une atrophie de la racine du poil, favorisant ainsi son extraction par le moyen classique de la cire froide, par exemple, ou même, dans certains cas, par un simple lavage. Plusieurs séances sont nécessaires pour produire une atrophie définitive du poil.

La multiplicité des petites cavités et des petits points d'émission électromagnétiques dans la grande surface de la sonde flexible permet une micro-action sur une grande quantité du mélange gel/produit pendant une durée suffisante pour favoriser l'éclatement du gel/produit par la liquéfaction du gel et l'évaporation de micro-particules. Cette micro-action serait d'autant plus efficace que la dimension des cavités et des points d'émission est petite, et a pour effet d'augmenter l'efficacité de pénétration du produit.

Pour la repousse des cheveux, le même procédé d'application sert à faire pénétrer le produit traitant dans la racine du cheveu et augmente son efficacité. Ce procédé s'applique non seulement à la repousse des cheveux naturels du cuir chevelu mais également aux cheveux transplantés pour augmenter l'efficacité du transplant.

Dans un procédé d'épilation définitive amélioré, avant l'application du mélange, on tond les poils d'une surface de la peau à traiter, laissant le bout des poils à fleur de la surface de la peau, dépassant par exemple d'environ 1 mm. Pour ceci, on utilise une tondeuse classique; le rasage n'est pas recommandé. On applique ensuite le mélange gel/produit et le flux d'énergie électromagnétique de haute fréquence, de préférence utilisant une sonde flexible alvéolée selon cette invention. Le traitement a pour effet d'amener le produit traitant jusqu'aux racines des poils tondus. On répète ce traitement après un temps de repos, par exemple de quelques jours, voire une semaine, éventuellement sans retonte. Ce traitement est poursuivi jusqu'à ce que les racines des poils s'atrophient complètement et, de préférence, que les poils, avec leur racine, tombent naturellement, par exemple lors du lavage. Ce procédé aboutit à une épilation définitive après moins de séances. De plus, les clientes d'un salon esthétique peuvent quitter le salon, dès la première séance, avec la peau nette, quand bien même le traitement n'est par terminé.

Les sondes flexibles 10,30,40 comportent chacune un grand nombre de points ou zones 16 conducteurs d'émission focalisée entrant en contact avec le gel chargé. On peut appliquer le gel contre la peau puis installer la sonde flexible 10,30,40. De préférence, cependant, on applique le gel contre la semelle 14 de la sonde, puis on applique la semelle de la sonde flexible contre la peau recouverte ou non du mélange. Pendant le traitement, il est possible d'immobiliser la sonde flexible contre la peau en utilisant une feuille de matière plastique qui sert à maintenir une bonne humidité pour toute la durée du traitement.

Les points ou zones 16 accessibles par les ouvertures 15 forment des fenêtres de conduction électrique et électromagnétique vers le gel conducteur et également des cavités pour recevoir le mélange gel/produit. Ces points ou zones 16 sont de préférence alignés en une ou plusieurs rangées le long de la semelle rectangulaire 14, afin de former une surface d'appui alvéolaire. D'autres dispositions sont possibles, mais la forme avec plusieurs rangées de points ou zones 16 a l'avantage de permettre le traitement de grandes surfaces, tout en évitant des interférences éventuelles, surtout si l'alimentation est par balayage sectoriel.

Pour permettre une action efficace sur les différentes parties du corps et sur les différentes sortes de pilosité, plusieurs sondes flexibles 10,30,40 de formes et/ou de dimensions semblables ou différentes peuvent être fixées à choix et de manière amovible sur différentes parties du corps, ces sondes étant de préférence reliées par paires à la sortie active et à la sortie indifférente du circuit générateur haute fréquence.

Il est également possible de prévoir des sondes rigides maniables selon WO 93/04636 ainsi qu'un embout à aiguille, interchangeables avec les sondes flexibles, permettant l'utilisation de sondes de formes spéciales pour les parties du corps délicates ainsi que, dans certains cas, du procédé classique d'épilation haute fréquence à aiguille, utilisant le même circuit d'alimentation haute fréquence.

## Revendications

1. Sonde (10,30,40) pour l'application d'un flux d'énergie électromagnétique haute fréquence sur la peau, la sonde étant pourvue d'une surface d'appui non-conductrice (14), destinée à être appliquée sur la peau, cette surface présentant une pluralité de points ou zones conducteurs (16) d'émission électromagnétique distincts formés par des parties de spires d'une bobine (12;12A,12B,12C,12D) disposées en retrait par rapport à cette surface,
caractérisée en ce qu'elle se présente sous forme d'une feuille flexible susceptible de se conformer à la partie du corps contre laquelle elle est appliquée, cette feuille flexible comportant des parties inférieure/interne et supérieure/externe non-conductrices, au moins ladite partie inférieure présentant ladite surface d'appui (14), laquelle est dotée de cavités (15), et en ce que la bobine (12;12A,12B,12C,12D) présente des spires de forme aplatie disposées en sandwich entre ladite partie inférieure et ladite partie supérieure avec les points ou zones d'émission (16) disposé(e)s au fond desdites cavités (15) dans la surface d'appui (14).

2. Sonde flexible (10) selon la revendication 1, comportant une bobine (12) dont les spires aplaties occupent la quasi-totalité de la surface de la feuille flexible, excepté le pourtour non-conducteur (17) de la feuille.

3. Sonde flexible (30,40) selon la revendication 1, comportant plusieurs bobines (12A,12B,12C,12D) dont les spires aplaties occupent la quasi-totalité de la surface de la feuille flexible, excepté le pourtour non-conducteur (17) de la feuille.

4. Sonde flexible (40) allongée selon la revendication 3, comportant plusieurs bobines (12A,12B,12C,12D) à spires aplaties disposées l'une à la suite de l'autre dans le sens de la longueur de la sonde (40).

5. Sonde flexible selon l'une quelconque des revendications précédentes, dont les cavités (15) varient en grandeur sur la surface d'appui (14) de la sonde.

6. Sonde flexible selon l'une quelconque des revendications précédentes, dont les points ou zones conducteurs d'émission électromagnétique distincts (16) sont formé(e)s par des parties exposées de spires de la ou les bobine(s) applatie(s) (12;12A,12B,12C,12D) débouchant par des orifices (15) dans cette surface, formant lesdites cavités.

7. Sonde flexible selon l'une quelconque des revendications précédentes, comportant une surface d'appui (14) sur ses deux faces.

8. Sonde flexible selon la revendication 7, où chaque face présente des cavités (15) de formes et/ou de dimensions et/ou de distribution différente(s) de celles de l'autre face.

9. Sonde flexible selon l'une quelconque des revendications précédentes, dont les cavités (15) dans la ou les surface(s) d'appui (14) sont de préférence alignées par rangées, formant une surface d'appui alvéolaire.

10. Procédé de traitement non-thérapeutique de la peau, notamment un procédé d'épilation de longue durée ou un procédé de traitement cosmétique de la calvitie utilisant une sonde flexible selon l'une quelconque des revendications précédentes, caractérisé en ce que l'on applique ladite surface d'appui (14) sur la peau à traiter, en ayant au préalable recouvert ladite surface d'appui (14) et/ou la peau à traiter de gel conducteur chargé d'un produit de traitement cosmétique bio-actif, pour amener le produit à pénétrer dans les follicules pileux de la peau jusqu'aux racines des poils par l'émission d'un flux d'énergie haute fréquence émis par lesdits points ou zones (16).

11. Procédé d'épilation cosmétique de longue durée selon la revendication 10, caractérisé en ce que l'on applique ladite surface d'appui (14) sur la peau à traiter, en ayant au préalable recouvert ladite surface d'appui (14) et/ou la peau à traiter de gel conducteur chargé d'un produit à effet d'atrophie sur les racines des poils, pour amener le produit à pénétrer dans les follicules pileux de la peau jusqu'aux racines des poils par l'émission d'un flux d'énergie haute fréquence émis par lesdits points ou zones (16), les poils tombant naturellement et/ou étant ensuite enlevés par des moyens idoines.

12. Procédé d'épilation selon la revendication 11, caractérisé en ce que, avant l'application du gel conducteur chargé sur la peau, on tond les poils de la surface de la peau à traiter, laissant le bout des poils à fleur de la surface de la peau, on applique ensuite le gel chargé et on applique le flux d'énergie électromagnétique de haute fréquence pour amener ledit produit jusqu'aux racines des poils tondus, et on répète ce traitement après un temps de repos, éventuellement sans retonte, jusqu'à ce que les racines des poils s'atrophient complètement et, de préférence, que les poils, avec leur racine, tombent naturellement.

13. Procédé de traitement cosmétique de la calvitie selon la revendication 10, caractérisé en ce que l'on applique ladite surface d'appui (14) sur le cuir chevelu à traiter, en ayant au préalable recouvert ladite surface d'appui (14) et/ou le cuir chevelu de gel conducteur chargé d'un produit régénérateur des cheveux, pour amener le produit à pénétrer dans les follicules pileux du cuir chevelu jusqu'aux racines des cheveux par l'émission haute fréquence émise par lesdits points ou zones (16).

14. Procédé selon l'une quelconque des revendications 10 à 13, caractérisé en ce qu'on dépose ledit gel chargé dans les cavités (15) ménagées dans la surface (14) de la sonde flexible, et on applique la surface (14) de la sonde flexible recouverte de mélange sur la peau.

## Claims

1. An applicator probe (10,30,40) for applying a flux of high-frequency electromagnetic energy to the skin, the applicator probe being provided with a non-conducting contact surface (14) adapted to be applied to the skin, this surface having a plurality of discrete conductive electromagnetic emissive points or areas (16) formed by parts of turns of a coil (12;12A,12B,12C,12D) which are set back in relation to this surface,
characterized in that it is in the form of a flexible sheet able to conform to the part of the body to which it is applied, this sheet comprising non-conductive lower/internal and upper/external parts, said contact surface (14) being present at least on said lower part and being provided with cavities (15), and in that the coil (12;12A,12B,12C,12D) has generally flat turns arranged in sandwich configuration between said lower part and said upper part with the emissive points or areas (16) arranged at the bottom of said cavities (15) in the contact surface (14).

2. A flexible applicator probe (10) according to claim 1, comprising a coil (12) whose generally flat turns occupy substantially the entire surface of the flexible sheet, except for a non-conducting periphery (17) of the sheet.

3. A flexible applicator probe (30,40) according to claim 1, comprising several coils (12A,12B,12C,12D) whose generally flat turns occupy substantially the entire surface of the flexible sheet, except for a non-conducting periphery (17) of the sheet.

4. An elongated flexible applicator probe (40) according to claim 3, comprising several coils (12A,12B,12C,12D) with generally flat turns arranged one after another along the length of the applicator probe (40).

5. A flexible applicator probe according to any one of the preceding claims, having cavities (15) of varying size over the contact surface (14) of the applicator probe.

6. A flexible applicator probe according to any one of the preceding claims, whose discrete conductive electromagnetic emission points or areas (16) are formed by exposed parts of the turns of the generally flat coil(s) (12;12A,12B,12C,12D) which open into cavity-forming orifices (15) in this surface.

7. A flexible applicator probe according to any one of the preceding claims, comprising a contact surface (14) on each of its two faces.

8. A flexible applicator probe according to claim 7, wherein each face has cavities (15) whose shapes and/or dimensions and/or distribution is/are different from those on the opposite face.

9. A flexible applicator probe according to any one of the preceding claims, whose cavities (15) in the contact surface(s) (14) are preferably aligned in rows, forming an alveolar contact surface.

10. A non-therapeutic treatment of the skin, in particular a long-lasting depilation method or a method for the cosmetic treatment of baldness, using a flexible applicator probe according to any one of the preceding claims, characterized in that said contact surface (14) is applied to the skin to be treated, having previously covered said contact surface (14) and/or the skin to be treated with conductive gel loaded with a cosmetic bio-active treatment product, to cause the product to penetrate into the pores of the skin down to the hair roots by the high-frequency flux of energy emitted by said points or areas (16).

11. A method of long lasting cosmetic depilation, using a flexible applicator probe according to claim 10, characterised in that said contact surface (14) is applied to the skin to be treated, having previously covered said contact surface (14) and/or the skin to be treated with conductive gel loaded with a product able to atrophy the hair roots, to cause the product to penetrate into the pores of the skin down to the hair roots by the high-frequency flux of energy emitted by said points or areas (16), the hair naturally dropping and/or being removed afterwards by suitable means.

12. A depilation method according to claim 11, characterized in that before applying the loaded conductive gel to the skin, the hairs over an area of the skin to be treated are cut, leaving the severed ends of the hairs flush with the surface of the skin, the loaded gel and the flux of high-frequency electromagnetic energy are then applied to cause the product to penetrate down to the roots of the cut hairs, and the treatment is repeated after a rest period, possibly without cutting the hairs again, until the hair roots have completely atrophied and the hairs with their roots preferably fall out naturally.

13. A method for the cosmetic treatment of baldness according to claim 10, characterised in that said contact surface (14) is applied to the scalp to be treated, having previously covered said contact surface (14) and/or the scalp with conductive gel loaded with a hair-regenerating product, to cause the product to penetrate into the hair follicles of the scalp down to the hair roots by the high-frequency flux of energy emitted by said points or areas (16).

14. A method according to any one of claims 10 to 13, characterized in that said loaded gel is applied into cavities (15) provided in the surface (14) of the flexible applicator probe, and the surface (14) of the flexible applicator probe covered with the mixture is applied to the skin.

## Patentansprüche

1. Sonde (10, 30, 40) zum Anlegen eines hochfrequenten elektromagnetischen Energieflusses an die Haut, wobei die Sonde mit einer nichtleitenden Auflagefläche (14) ausgestattet ist, die dazu bestimmt ist, auf die Haut aufgelegt zu werden, und eine Vielzahl von diskreten, leitenden Punkten oder Bereichen (16) elektromagnetischer Ausstrahlung aufweist, die von Abschnitten von Windungen einer Spule (12; 12A, 12B, 12C, 12D) gebildet werden, die gegenüber dieser Auflagefläche zurückgezogen angeordnet sind,
dadurch gekennzeichnet, dass sie sich in Gestalt einer biegsamen Folie darbietet, die fähig ist, sich an die Körperpartie anzupassen, auf die sie aufgelegt ist, wobei diese biegsame Folie einen unteren/inneren und einen oberen/äusseren, nichtleitenden Abschnitt umfasst und zumindest der benannte untere Abschnitt die benannte Auflagefläche (14) aufweist, die mit Vertiefungen (15) versehen ist, und dadurch, dass die Spule (12; 12A, 12B, 12C, 12D) Windungen abgeplatteter Form aufweist, die zwischen dem benannten unteren und dem benannten oberen Abschnitt sandwichartig angeordnet sind, wobei die Punkte oder Bereiche der Ausstrahlung (16) am Boden der benannten Vertiefungen (15) in der Auflagefläche (14) angeordnet sind.

2. Biegsame Sonde (10) gemäss Anspruch 1 mit einer Spule (12), deren abgeplattete Windungen mit Ausnahme der nichtleitenden Peripherie (17) der Folie fast die gesamte Oberfläche der biegsamen Folie einnehmen.

3. Biegsame Sonde (30, 40) gemäss Anspruch 1 mit mehreren Spulen (12A, 12B, 12C, 12D), deren abgeplattete Windungen mit Ausnahme der nichtleitenden Peripherie (17) der Folie fast die gesamte Oberfläche der biegsamen Folie einnehmen.

4. In die Länge gezogene, biegsame Sonde (40) gemäss Anspruch 3 mit mehreren Spulen (12A, 12B, 12C, 12D) mit abgeplatteten Windungen, die in Längsrichtung der Sonde (40) aufeinanderfolgend angeordnet sind.

5. Biegsame Sonde gemäss einem beliebigen der voraufgehenden Ansprüche, deren Vertiefungen (15) in ihrer Grösse über die Auflagefläche (14) der Sonde variieren.

6. Biegsame Sonde gemäss einem beliebigen der voraufgehenden Ansprüche, deren diskrete, leitende Punkte oder Bereiche (16) elektromagnischer Ausstrahlung durch die exponierten Abschnitte der Windungen der abgeplatteten Spule(n) (12; 12A, 12B, 12C, 12D) gebildet werden, die durch Löcher (15), die die benannten Vertiefungen bilden, in diese Auflagefläche austreten.

7. Biegsame Sonde gemäss einem beliebigen der voraufgehenden Ansprüche mit einer Auflagefläche (14) auf beiden ihrer Seiten.

8. Biegsame Sonde gemäss Anspruch 7, wo jede Seite Vertiefungen (15) in Formen und/oder Abmessungen und/oder einer Verteilung aufweist, die sich von denen der anderen Seite unterscheiden.

9. Biegsame Sonde gemäss einem beliebigen der voraufgehenden Ansprüche, deren Vertiefungen (15) in der oder den Auflagefläche(n) (14) bevorzugt reihenweise angeordnet sind und eine zellenförmige Auflagefläche bilden.

10. Nichttherapeutisches Verfahren zur Behandlung der Haut, namentlich ein Verfahren zur lang anhaltenden Haarentfernung oder ein Verfahren zur kosmetischen Behandlung der Kahlköpfigkeit, das eine biegsame Sonde gemäss einem beliebigen der voraufgehenden Ansprüche verwendet und dadurch gekennzeichnet ist, dass man die benannte Auflagefläche (14) auf die zu behandelnde Haut auflegt, wobei im voraus die benannte Auflagefläche (14) und/oder die zu behandelnde Haut mit leitfähigem Gel bedeckt worden ist, das mit einem bioaktiven Produkt für die kosmetische Behandlung beladen ist, um das Produkt durch die Ausstrahlung eines Flusses hochfrequenter Energie, der von den benannten Punkten oder Bereichen (16) ausgeht, dazu zu bringen, in den Haarbälgen der Haut bis zu den Haarwurzeln vorzudringen.

11. Verfahren zur lang anhaltenden kosmetischen Haarentfernung gemäss Anspruch 10, dadurch gekennzeichnet, dass man die benannte Auflagefläche (14) auf die zu behandelnde Haut auflegt, wobei im voraus die benannte Auflagefläche (14) und/oder die zu behandelnde Haut mit leitfähigem Gel bedeckt worden ist, das mit einem Produkt mit atrophischer Wirkung auf die Haarwurzeln beladen ist, um das Produkt durch die Ausstrahlung eines Flusses hochfrequenter Energie, der von den benannten Punkten oder Bereichen (16) ausgeht, dazu zu bringen, in den Haarbälgen der Haut bis zu den Haarwurzeln vorzudringen, wobei die Haare natürlich ausfallen und/oder in der Folge durch passende Mittel entfernt werden.

12. Verfahren zur Haarentfernung gemäss Anspruch 11, dadurch gekennzeichnet, dass man vor Aufbringen des beladenen, leitfähigen Gels auf die Haut die Haare von der Oberfläche der zu behandelnden Haut wegschert, wobei die Haarenden bis zur Hautoberfläche stehen bleiben, dass man dann das beladene Gel aufbringt und den Fluss hochfrequenter elektromagnetischer Energie anlegt, um das benannte Produkt bis zu den Wurzeln der abgeschorenen Haare zu bringen, und diese Behandlung nach einer Erholungszeit und gegebenenfalls ohne nochmaliges Abscheren wiederholt, bis die Haarwurzeln völlig geschwunden sind und bis vorzugsweise die Haare mit ihren Wurzeln natürlich ausfallen.

13. Verfahren zur kosmetischen Behandlung der Kahlköpfigkeit gemäss Anspruch 10, dadurch gekennzeichnet, dass man die benannte Auflagefläche (14) auf die zu behandelnde Kopfhaut auflegt, wobei im voraus die benannte Auflagefläche (14) und/oder die Kopfhaut mit leitfähigem Gel bedeckt worden ist, das mit einem Produkt zur Haarregenerierung beladen ist, um das Produkt durch die hochfrequente Ausstrahlung, die von den benannten Punkten oder Bereichen (16) ausgeht, dazu zu bringen, in den Haarbälgen der Kopfhaut bis zu den Haarwurzeln vorzudringen.

14. Verfahren gemäss einem beliebigen der Ansprüche 10 bis 13, dadurch gekennzeichnet, dass man das benannte beladene Gel in die in der Auflagefläche (14) der biegsamen Sonde eingelassenen Vertiefungen (15) einbringt und die mit der Mischung bedeckte Auflagefläche (14) der biegsamen Sonde auf die Haut auflegt.
